(19) Europäisches Patentamt
European Patent Office
Office européen des brevets

(11) **EP 3 206 699 B1**

(12) **EUROPEAN PATENT SPECIFICATION**

(45) Date of publication and mention
of the grant of the patent:
**13.09.2023 Bulletin 2023/37**

(21) Application number: **15778924.9**

(22) Date of filing: **09.10.2015**

(51) International Patent Classification (IPC):
*A61K 35/20* (2006.01)   *A61K 38/01* (2006.01)
*A61P 21/00* (2006.01)   *A61K 31/12* (2006.01)
*A61K 31/7048* (2006.01)   *A61K 31/202* (2006.01)
*A61K 31/352* (2006.01)   *A23L 33/00* (2016.01)
*A23L 2/00* (2006.01)

(52) Cooperative Patent Classification (CPC):
(C-Sets available)
**A61K 35/20; A23L 2/39; A23L 2/52; A23L 2/66;
A23L 33/105; A23L 33/185; A23L 33/19;
A61K 31/12; A61K 31/202; A61K 31/352;
A61K 31/7048; A61K 38/018; A61P 21/00;**
A23V 2002/00; A23V 2200/00         (Cont.)

(86) International application number:
**PCT/EP2015/073361**

(87) International publication number:
**WO 2016/058919 (21.04.2016 Gazette 2016/16)**

(54) **IMPROVEMENT IN MUSCLE FUNCTIONALITY OF ELDERLY MALES**

VERBESSERUNG DER MUSKULÄREN FUNKTIONALITÄT IN ÄLTEREN MÄNNLICHEN PATIENTEN

AMÉLIORATION DE LA FONCTIONNALITÉ MUSCULAIRE D'HOMMES ÂGÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.10.2014 US 201462063752 P**

(43) Date of publication of application:
**23.08.2017 Bulletin 2017/34**

(73) Proprietor: **Société des Produits Nestlé S.A.
1800 Vevey (CH)**

(72) Inventors:
• **BREUILLE, Denis
CH-1010 Lausanne (CH)**
• **MORITANI, Toshio
Kyoto 606-8501 (JP)**
• **VINYES PARES, Gerard
E-08510 Barcelona (ES)**

(74) Representative: **Chautard, Cécile
Société des Produits Nestlé S.A.
Avenue Nestlé 55
1800 Vevey (CH)**

(56) References cited:
**WO-A1-2011/011252   WO-A1-2012/143402
WO-A1-2013/053795   WO-A1-2014/170245**

• **MAGNE H ET AL: "Nutritional strategies to counteract muscle atrophy caused by disuse and to improve recovery.", NUTRITION RESEARCH REVIEWS 2013 CLERMONT UNIVERSITE, UNIVERSITE D'AUVERGNE, UNITE DE NUTRITION HUMAINE, BP 10448, F-63000, CLERMONT-FERRAND, FRANCE. E-MAIL HUGUES.MAGNE@CLERMONT.INRA.FR, vol. 26, no. 2, 9 August 2013 (2013-08-09) , - 9 August 2013 (2013-08-09), page 149, XP009187181,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 206 699 B1

(52) Cooperative Patent Classification (CPC): (Cont.)

C-Sets
**A61K 31/12, A61K 2300/00;**
**A61K 31/202, A61K 2300/00;**
**A61K 31/352, A61K 2300/00;**
**A61K 31/7048, A61K 2300/00;**
**A61K 38/018, A61K 2300/00;**
A23V 2002/00, A23V 2200/00, A23V 2200/02,
A23V 2200/306, A23V 2250/2116,
A23V 2250/2117, A23V 2250/54252

**Description**

BACKGROUND

The present disclosure generally relates to a composition comprising a protein source and an antioxidant for administration to an elderly male. More specifically, the present disclosure relates to administering to an elderly male a composition comprising a protein source and an antioxidant to treat or prevent sarcopenia, reduce a loss of muscle functionality (e.g. muscle strength, gait speed, etc.), increase muscle functionality after muscle atrophy, and/or improve recovery of muscle functionality after muscle atrophy.

Sarcopenia is defined as the age-associated loss of muscle mass and functionality (including muscle strength and gait speed). Muscle functionality and physical ability decline with the loss of muscle mass. Impaired muscle functionality is highly predictive of the incidence of immobility, disability, and mortality in advanced age. With the rising elderly population, sarcopenia becomes increasingly prevalent such that 45% of the elderly U.S. population has moderate-to-severe symptoms. The U.S. health care direct and indirect costs attributable to sarcopenia reach nearly $19 billion. Therefore, prevention and/or treatment of sarcopenia would have a great impact on the health and quality of life of our society and consequently on the economy associated with health care. Unfortunately, the etiology and the physiopathological mechanism of sarcopenia are still poorly understood, making effective measures for prevention or treatment difficult.

One of the main hypotheses developed to explain the progressive muscle loss observed with aging is a decreased anabolic effect of meal ingestion due to a lower stimulation of muscle protein synthesis by the nutrients. This hypothesis is called muscle anabolic resistance. In addition, oxidative stress and/or a low grade inflammation have also been demonstrated to be associated with frailty in the elderly and could be partly responsible for anabolic resistance either directly or through a decreased sensitivity of muscle to insulin.

SUMMARY

Without being bound by theory, the inventors surprisingly found that efforts to reduce sarcopenia in an elderly male are improved when the elderly male is fed with a nutritional composition comprising a protein source and at least one antioxidant. For example, such a composition can reduce loss of muscle functionality (e.g. muscle strength, gait speed, etc.) or improve muscle functionality in an elderly male after muscle atrophy who is administered the composition, relative to a diet lacking such a composition.

The composition comprises a fatty acid. The fatty acid can be an n-3 fatty acid.

The antioxidant comprises a polyphenol selected from the group consisting of curcumin, rutin, quercetin and combinations thereof.

The protein source comprises whey protein.

In an embodiment, the protein source comprises a protein selected from the group consisting of casein, pea protein, soy protein and combinations thereof.

In an embodiment, the composition comprises an n-3 fatty acid in addition to the protein source.

In an embodiment, the composition is administered at least twice a week for a time period of at least one month.

In an embodiment, the muscle functionality comprises a characteristic selected from the group consisting of muscle strength, gait speed, and combinations thereof.

In an embodiment, the muscle functionality is in a skeletal muscle selected from the group consisting of gastrocnemius, tibialis, soleus, extensor digitorum longus (EDL), biceps femoris, semitendinosus, semimembranosus, gluteus maximus, and combinations thereof.

In an embodiment, the composition is administered in an amount that provides 0.1 to 0.4 g of the protein source per kg body weight of the elderly male per day.

In an embodiment, the composition is administered in an amount that provides 0.01 to 0.04 g of leucine per kg body weight of the elderly male per day.

In an embodiment, the elderly male has sarcopenia.

In another embodiment, the present disclosure provides a composition comprising a protein source, an antioxidant and a fatty acid in an amount that is therapeutically effective for use in at least one of: (i) treating sarcopenia in an elderly male having sarcopenia, (ii) preventing sarcopenia in an elderly male, (iii) reducing a loss of muscle functionality in an elderly male in an elderly male having sarcopenia.

In an embodiment, the composition comprises an n-3 fatty acid in addition to the protein source.

In an embodiment, the composition comprises the protein source in an amount from 0.2-100% based on dry weight of the composition.

In an embodiment, the protein source comprises leucine, and the leucine is present in the composition up to an amount of 10 wt%.

In an embodiment, the composition is selected from the group consisting of food compositions, dietary supplements, nutritional compositions, nutraceuticals, powdered nutritional products to be reconstituted in water or milk before consumption, food additives, medicaments, drinks, and combinations thereof.

In another embodiment, the present disclosure provides a composition for use in preventing sarcopenia in an elderly male comprising administering a composition comprising a protein source, an antioxidant and a fatty acid to an elderly male at risk thereof.

In an embodiment, the composition comprises an n-3 fatty acid in addition to the protein source.

An advantage of the present disclosure is to provide a composition, such as a food product or a food supplement, that treats sarcopenia in elderly males.

Another advantage of the present disclosure is to provide a composition, such as a food product or a food supplement, that prevents sarcopenia.

Still another advantage of the present disclosure is to provide a composition, such as a food product or a food supplement, that reduces a loss of muscle functionality (e.g. muscle strength, gait speed, etc.) in elderly males, relative to the loss that would be experienced during consumption of a diet lacking the composition.

An additional advantage of the present disclosure is to provide a composition, such as a food product or a food supplement, that increases muscle functionality (e.g. muscle strength, gait speed, etc.) in elderly males, relative to the muscle functionality (e.g. muscle strength, gait speed, etc.) that would be present from consumption of a diet lacking the composition.

Another advantage of the present disclosure is to provide a composition, such as a food product or a food supplement, that improves recovery of muscle functionality (e.g. muscle strength, gait speed, etc.) after muscle atrophy in elderly males, relative to the recovery that would be present from consumption of a diet lacking the composition.

Yet another advantage of the present disclosure is to beneficially promote reduction, prevention, or treatment of sarcopenia in elderly males.

Another advantage of the present disclosure is to provide nutritional strategies to reduce development of sarcopenia in elderly males, especially to reduce loss of muscle functionality (e.g. muscle strength, gait speed, etc.) in elderly frail males.

Additional features and advantages are described in, and will be apparent from, the following Detailed Description and the Figures.

BRIEF DESCRIPTION OF THE DRAWINGS

FIG. 1 is a schematic diagram showing the method for estimating cross sectional area of muscle used in the experimental example disclosed herein.

FIG. 2 is a table of data from the experimental example disclosed herein.

FIG. 3 is a graph showing changes in left knee extension strength in the experimental example disclosed herein.

FIG. 4 is a graph showing boxplots of left and right knee extension strength by visit and gender and treatment group in the experimental example disclosed herein.

FIG. 5 is a graph showing plots showing mean +/- SD for left and right knee extension strength by visit and treatment group in the experimental example disclosed herein.

DETAILED DESCRIPTION

All percentages are by weight of the total weight of the composition unless expressed otherwise. Similarly, all ratios are by weight unless expressed otherwise. When reference is made to the pH, values correspond to pH measured at 25 °C with standard equipment. As used herein, "about" is understood to refer to numbers in a range of numerals, for example the range of -10% to +10% of the referenced number, preferably -5% to +5% of the referenced number, more preferably -1% to +1% of the referenced number, most preferably -0.1% to +0.1% of the referenced number.

Furthermore, all numerical ranges herein should be understood to include all integers, whole or fractions, within the range. Moreover, these numerical ranges should be construed as providing support for a claim directed to any number or subset of numbers in that range. For example, a disclosure of from 1 to 10 should be construed as supporting a range of from 1 to 8, from 3 to 7, from 1 to 9, from 3.6 to 4.6, from 3.5 to 9.9, and so forth.

As used herein and in the appended claims, the singular form of a word includes the plural, and vice versa, unless the context clearly dictates otherwise. Thus, the references "a," "an" and "the" are generally inclusive of the plurals of the respective terms. For example, reference to "an ingredient" or "a method" includes a plurality of such "ingredients" or "methods." The term "and/or" used in the context of "X and/or Y" should be interpreted as "X," or "Y," or "X and Y."

Similarly, the words "comprise," "comprises," and "comprising" are to be interpreted inclusively rather than exclusively. Likewise, the terms "include," "including" and "or" should all be construed to be inclusive, unless such a construction is clearly prohibited from the context. However, the embodiments provided by the present disclosure may lack any element that is not specifically disclosed herein. Thus, a disclosure of an embodiment defined using the term "comprising" is also a disclosure of embodiments "consisting essentially of" and "consisting of" the disclosed components. Where used herein, the term "example," particularly when followed by a listing of terms, is merely exemplary and illustrative, and should not be deemed to be exclusive or comprehensive. Any embodiment disclosed herein can be combined with any other embodiment disclosed herein unless explicitly indicated otherwise.

The term "elderly" means a person above the age of 60 years, preferably above 63 years, and more preferably above 65 years. The term "frail" refers to a person which is physically weak, i.e. not strong, but fragile.

The terms "treatment" and "treating" include any effect that results in the improvement of the condition or disorder, for example lessening, reducing, modulating, or eliminating the condition or disorder. Non-limiting examples of "treating" or "treatment of" a condition or disorder include: (1) inhibiting the condition or disorder, i.e. arresting the development of the condition or disorder or its clinical symptoms and (2) relieving the condition or disorder, i.e. causing the temporary or permanent regression of the condition or disorder or its clinical symptoms.

The terms "prevention" or "preventing" mean causing the clinical symptoms of the referenced condition or disorder to not develop in an individual that may be exposed or predisposed to the condition or disorder but does not yet experience or display symptoms of the condition or disorder. The terms "condition" and "disorder" mean any disease, condition, symptom, or indication.

The terms "food," "food product" and "food composition" mean a product or composition that is intended for ingestion by a human and provides at least one nutrient to the human. The compositions of the present disclosure, including the many embodiments described herein, can comprise, consist of, or consist essentially of the essential elements and limitations described herein, as well as any additional or optional ingredients, components, or limitations described herein or otherwise useful in a diet for elderly males.

As used herein, "complete nutrition" contains sufficient types and levels of macronutrients (protein, fats and carbohydrates) and micronutrients to be sufficient to be a sole source of nutrition for the animal to which the composition is administered. Individuals can receive 100% of their nutritional requirements from such complete nutritional compositions.

An aspect of the present disclosure is a composition comprising a protein source, a fatty acid and an antioxidant for use in the treatment or prevention of sarcopenia, reducing a loss of muscle functionality (e.g. muscle strength, gait speed, etc.), increasing muscle functionality after muscle atropy (e.g. muscle strength, gait speed, etc.), and/or improving recovery of muscle functionality (e.g. muscle strength, gait speed, etc.) after muscle atropy in elderly males. Another aspect of the present disclosure is a composition for use in a therapeutically effective amount comprising a protein source, a fatty acid and an antioxidant to an elderly male to treat the elderly male for sarcopenia, prevent sarcopenia in the elderly male, reduce a loss of muscle functionality (e.g. muscle strength, gait speed, etc.) in the elderly male, increase the muscle functionality after muscle atrophy (e.g. muscle strength, gait speed, etc.) in the elderly male, and/or improve recovery of muscle functionality (e.g. muscle strength, gait speed, etc.) after muscle atrophy in the elderly male.

Muscle atrophy, as treated or prevented according to the present disclosure, may be caused by many reasons. For example, it may result from lack of physical activity, such as from immobilization or low physical activity associated with aging (sarcopenia associated with aging process), hip-fracture recovery, or several co-morbidities of diseases, such as cancer, AIDS, congestive heart failure, COPD (chronic obstructive pulmonary disease), renal failure, trauma, sepsis, and severe burns, for example. Muscle atrophy may also result from insufficient or inappropriate nutrition or starvation. Very commonly, muscle atrophy results from disuse or insufficient use of the respective muscle.

The muscle referred to in the present disclosure is preferably a skeletal muscle. For example, the composition disclosed herein may be used to reduce the loss of muscle functionality in the arms and/or the legs of the elderly male. The muscle may be one or more of the following: gastrocnemius, tibialis, soleus, extensor, digitorum longus (EDL), biceps femoris, semitendinosus, semimembranosus, or gluteus maximus.

Muscle atrophy may result in the disorder of sarcopenia, i.e. lost muscle mass, size, and functionality because of aging. The muscle atrophy may be of different grades, such as severe muscle atrophy as in extreme frailty of elderly persons. Extremely frail elderly persons can have difficulty in every-day activities and taking care of themselves. Muscle atrophy of a less severe degree will allow some movement and some muscle activity, but the muscle activity is insufficient to sustain the complete muscle tissue. The mechanisms involved in treating or preventing age-associated sarcopenia are different from treating or preventing loss of muscle function in younger persons.

The composition disclosed herein comprises a protein source , fatty acid and an antioxidant and can reduce loss of muscle functionality and/or improve muscle functionality in an elderly male who is administered the composition, relative to a diet lacking such a composition. In an embodiment, the reduced loss of muscle functionality (e.g. muscle strength, gait speed, etc.), the improved muscle functionality (e.g. muscle strength, gait speed, etc.), the improved recovery of muscle functionality (e.g. muscle strength, gait speed, etc.) after muscle atrophy, the treatment of sarcopenia, and/or the prevention of sarcopenia is achieved without modification of muscle size or muscle mass.

The protein source comprises whey protein and can comprise a protein from animal or plant origin, for example milk proteins, soy proteins, and/or pea proteins. In a preferred embodiment, the protein source can comprise a protein selected from casein protein; pea protein; soy protein; wheat protein; corn protein; rice protein; proteins from legumes, cereals and grains; and combinations thereof. Additionally the protein source may comprise a protein from nuts and/or seeds. In an embodiment, the composition comprises protein in an amount of 0.2-100% based on dry weight, preferably 1-95% based on dry weight, more preferably 2-90% based on dry weight, even more preferably 3-80% based on dry weight, and most preferably 5-70% based on dry weight. In an embodiment, the composition is administered to the elderly male in a daily dose that provides 0.1 to 0.4 g of the protein per kg body weight of the elderly male, preferably 0.2 to 0.35 g of the protein per kg body weight of the elderly male.

The protein source comprises whey protein. The whey protein may be unhydrolyzed or hydrolyzed whey protein. The whey protein may be any whey protein, for example the whey protein can be selected from the group consisting of whey protein concentrates, whey protein isolates, whey protein micelles, whey protein hydrolysates, acid whey, sweet whey, modified sweet whey (sweet whey from which the caseino-glycomacropeptide has been removed), a fraction of whey protein, and any combination thereof. In a preferred embodiment, the whey protein comprises whey protein isolate and/or modified sweet whey.

As noted above, the protein source can comprise a protein from animal or plant origin, for example milk proteins, soy proteins, and/or pea proteins. In an embodiment, the protein source comprises casein. Casein may be obtained from any mammal but is preferably obtained from cow milk and preferably as micellar casein.

The composition can comprise one or more branched chain amino acids. For example, the composition can comprise leucine, isoleucine and/or valine. The protein source in the composition may comprise leucine in free form and/or leucine bound as peptides and/or proteins such as dairy, animal or vegetable proteins. In an embodiment, the composition comprises the leucine in an amount up to 10 wt% of the dry matter of the composition. Leucine can be present as D- or L-leucine and preferably the L-form. If the composition comprises leucine, the composition can be administered in a daily dose that provides 0.01 to 0.04 g of the leucine per kg body weight, preferably 0.02 to 0.035 g of the leucine per kg body weight. Such doses are particularly applicable to complete nutrition compositions, but one of ordinary skill will readily recognize how to adapt these doses for an oral nutritional supplement (ONS).

The antioxidant is selected from the group of polyphenols. Particularly preferred are food-grade polyphenols. A compound is considered "food-grade" if it is generally accepted and considered safe for food applications.

Mixtures of antioxidants may be used. For example, antioxidants may be provided as food compositions that are rich in antioxidants or as extracts thereof. A food composition that is "rich in antioxidants" has an ORAC (oxygen radical absorbance capacity) rating of at least 100 per 100 g of the composition.

Non-limiting examples of suitable vitamins include vitamin E (tocopherol), vitamin A (retinol or beta-carotene) and vitamin C (ascorbic acid). Non-limiting examples of suitable flavonoids are hesperetine-7-glucoside and catechin.

The polyphenol is selected from the group consisting of curcumin, rutin, quercetin and combinations thereof. Metabolites of antioxidants may be used. In a particularly preferred embodiment, the antioxidant is a combination of two or more antioxidants.

Cocoa, coffee and tea are high in antioxidants. Several spices or herbs high in antioxidants may be used, such as oregano, cumin, ginger, garlic, coriander, onion, thyme, marjoram, tarragon, peppermint, and/or basil. Fruit extracts or dried fruits may be used, for exampl pears, apples, raisins, grapes, figs, cranberries, blueberries, blackberries, raspberries, strawberries, blackcurrants, cherries, plums, oranges, mango, and/or pomegranates. The composition can comprise vegetables high in antioxidants, such as cabbage, broccoli, bettroot, artichoke heads, black olives, black beans, celery, onion, parsley and/or spinach.

The antioxidant may be a purified compound or a partially purified compound.

In an embodiment, the weight ratio of the protein source and the antioxidant is 40:1 to 1:1, such as from 35:1 to 2:1, preferably from 30:1 to 5:1, such as from 28:1 to 8:1, and even more preferably from 25:1 to 10:1.

In a preferred embodiment, the antioxidant comprises one or more polyphenols. Mixtures of polyphenols may be used, such as two or more polyphenols. Polyphenols may also be provided as food compositions rich in polyphenols or extracts thereof.

Cocoa, coffee and tea are high in polyphenols. Fruit extracts or dried fruits may be used as a source of polyphenols, for example pears, apples, grapes, cranberries, blueberries, blackberries, raspberries, strawberries, blackcurrants, cherries, plums, and/or pomegranates. Also some nuts and seeds are rich in polyphenols, such as chestnuts, hazel nuts and flaxseed. Non-limiting examples of vegetables high in polyphenols are cabbage, broccoli, beetroot, artichoke heads, black olives, black beans, celery, onions, parsley and spinach.

The polyphenol may be a purified compound or a partially purified compound. Non-limiting examples of suitable polyphenols are phenolic acids; flavonoids, such as flavonols, flavones, isoflavones, flavanones, anthocyanins, and flavanols; stilbenes; and lignans. The polyphenol is selected from the group consisting of curcumin, rutin, quercetin and combinations thereof.

In an embodiment, the composition comprises one or more whey proteins and one or more polyphenols in a weight ratio of 300:1 to 2:1, such as from 100:1 to 5:1, preferably from 60:1 to 10:1, even more preferably from 50:1 to 20:1.

The composition comprising a protein source and an antioxidant can be administered to an elderly male in a therapeutically effective dose. The therapeutically effective dose can be determined by the person skilled in the art and will depend on a number of factors known to those of skill in the art, such as the severity of the condition and the weight and general state of the elderly male.

The composition may be administered to an elderly male in an amount sufficient to prevent or at least partially reduce the risk of developing sarcopenia in instances where the condition of sarcopenia has yet not been developed in the elderly male. Such an amount is defined to be "a prophylactically effective dose." Again, the precise amounts depend on a number of factors relating to the elderly male, such as their weight, health and how much muscle functionality (e.g. muscle strength, gait speed, etc.) is being lost.

The composition is preferably administered as a supplement to the diet of an elderly male daily or at least twice a week. In an embodiment, the composition is administered to the elderly male consecutively for a number of days, preferably until an increase in muscle functionality (e.g. muscle strength, gait speed, etc.) relative to that before administration is achieved. For example, the composition can be administered to the elderly male daily for at least 30, 60 or 90 consecutive days. As another example, the composition can be administered to the elderly male for a longer period, such as a period of 1, 2, 3, 4, 5, 6, 7, 8, 9 or 10 years.

In a preferred embodiment, the composition is administered to the elderly male for at least 3 months, for example a period of 3 months to 1 year, and preferably for at least 6 months.

The above examples of administration do not require continuous daily administration with no interruptions. Instead, there may be some short breaks in the administration, such as a break of two to four days during the period of administration. The ideal duration of the administration of the composition can be determined by those of skill in the art.

In a preferred embodiment, the composition is administered to the elderly male orally or enterally (e.g. tube feeding). For example, the composition can be administered to the elderly male as a beverage, a capsule, a tablet, a powder or a suspension.

The composition can be any kind of composition that is suitable for human and/or animal consumption. For example, the composition may be selected from the group consisting of food compositions, dietary supplements, nutritional compositions, nutraceuticals, powdered nutritional products to be reconstituted in water or milk before consumption, food additives, medicaments, beverages and drinks. In an embodiment, the composition is an oral nutritional supplement (ONS), a complete nutritional formula, a pharmaceutical, a medical or a food product. In a preferred embodiment, the composition is administered to the elderly male as a beverage. The composition may be stored in a sachet as a powder and then suspended in a liquid such as water for use.

In some instances where oral or enteral administration is not possible or not advised, the composition may also be administered parenterally.

In an embodiment, the composition comprises whey protein in an amount of 0.5-100% based on dry weight of the composition. For example, the composition can be a nutritional supplement which is almost entirely whey protein. Thus, in a preferred embodiment, the composition comprises more than 60% whey protein based on dry weight, such as above 70% whey protein, preferably above 80% whey protein, such as above 85% whey protein, even more preferably above 90% whey protein, such as above 92% whey protein, in particular above 95% whey protein, such as above 97% whey protein based on dry weight.

In an embodiment, the composition is a nutritional supplement comprising a protein source, for example whey protein, and also other ingredients optimal for an elderly male to intake, such as one or more fatty acids, preferably essential fatty acids; proteins; carbohydrates; dietary fibers; vitamins; minerals; or probiotics. In such embodiments, the composition can comprise whey protein in an amount of 0.5-50% based on dry weight, such as 1-40% whey protein based on dry weight, preferably 2-35% whey protein, such as 3-30% whey protein, and more preferably 5-20% whey protein based on dry weight.

The amount of the protein source administered to the elderly male depends on both the weight and health of the elderly male, i.e. the severity of sarcopenia and/or the degree of loss of muscle functionality (e.g. muscle strength, gait speed, etc.) in the elderly male. In an embodiment, the composition is administered to the elderly male in an amount that provides 0.03 to 1.0 g of whey protein per kg of body weight per day, such as 0.05 to 0.7 g of whey protein per kg body weight per day, preferably about 0.1 to 0.5 g whey protein per kg body weight per day.

Thus, in an embodiment, the composition is administered to provide 5-50 g of whey protein per day, such as 12-40 g whey protein per day, preferably 15-30 g of whey protein per day, such as from 16-25 g of whey protein per day, even more preferably 20 g of whey protein per day. In an embodiment, a single serving of the composition provides 5-50 g of whey protein, such as 10-40 g of whey protein, and preferably 12-35 g of whey protein, such as 15-30 g whey protein.

The composition comprising a protein source and an antioxidant further comprises a fatty acid. The fatty acid may be any fatty acid and may be one or more fatty acids, such as a combination of fatty acids. The fatty acid preferably comprises an essential fatty acid, such as the essential polyunsaturated fatty acids, namely linoleic acid (C18:2n-3) and α-linolenic acid (C18:3n-3). The fatty acid may comprise long-chain polyunsaturated fatty acids, such as eicosapentaenoic acid (C20:5n-3), arachidonic acid (C20:4n-6), docosahexaenoic acid (C22:6n-3), or any combination thereof. In a preferred embodiment, the fatty acid comprises an n-3 (omega 3) fatty acid and/or an n-6 (omega 6) fatty acid. The fatty acid preferably comprises eicosapentaenoic acid.

The fatty acid may be derived from any suitable source containing fatty acids, such as coconut oil, rapeseed oil, soya oils, corn oil, safflower oil, palm oil, sunflower oil or egg yolk. The source of the fatty acid is preferably fish oil.

In some embodiments, the composition is administered to the elderly male in a single dosage form, i.e. all compounds are present in one product to be given to an elderly male in combination with a meal. In other embodiments, the composition is co-administered in separate dosage forms, for example the protein source separately from the antioxidant.

In an embodiment, the composition further comprises vitamin D.

The composition can be administered to the elderly male after electrical muscle stimulation (EMS), for example within two hours thereafter, preferably within one hour, and more preferably within thirty minutes. EMS may also be referred to as muscular electro-stimulation, muscular electric stimulation, neuromuscular electrical stimulation (NMES) or electromyostimulation, and these terms may be used interchangeably. Electrical muscle stimulation is the elicitation of muscle contraction using electric impulses. The impulses are generated by an apparatus and delivered through electrodes on the skin in direct proximity to the muscles to be stimulated. The impulses mimic the action potential coming from the central nervous system, causing the muscles to contract. The electrodes can be pads which adhere to the skin, but the electrodes may also be other forms.

EMS is preferably given to the elderly male at least once a week, preferably at least two times a week, and more preferably at least three times a week. Preferably the composition comprising a protein source and an antioxidant is administered at least twice a week and more preferably daily while the elderly male is on the EMS regimen. The elderly male can use an apparatus for physical stimulation or electrically muscle stimulation, for example an apparatus or machine forcing muscular functions to enhance energy loss.

EXAMPLE

The following example presents scientific data developing and supporting the concept of administering a composition comprising a protein source and an antioxidant to an elderly male to treat the elderly male for sarcopenia, prevent sarcopenia in the elderly male, reduce a loss of muscle functionality (e.g. muscle strength, gait speed, etc.) in the elderly male, increase the muscle functionality (e.g. muscle strength, gait speed, etc.) in the elderly male, and/or improve recovery of muscle functionality (e.g. muscle strength, gait speed, etc.) after muscle atrophy in the elderly male.

As detailed below, a pilot trial aimed to study the effects of a combined approach with electrical muscle stimulation (EMS) and a whey-based nutritional supplement (+/- polyphenols and PUFAs) on muscle size and functionality of physically compromised population. Forty-one frail subjects were randomized in a 1:1:1 ratio to one of the three study groups; Isocaloric (95kcal) beverage containing either (A) 20g of carbohydrate + placebo capsules (CHO), (B) 20g of whey protein isolate + placebo capsules (WHEY), or (C) 20g of whey protein isolate + rutin capsules (500mg rutin per day)+ w3-FA capsules (500mg curcumin and 1.5g w3-FA per day) (W-BIO). All subjects received EMS training twice a week for a period of 12 weeks. For the primary outcome, results indicated that the differences in muscle thickness (in mm) and muscle cross section area (in mm$^2$) for the calf and thigh among three groups at week 12 after the start of treatment were not statistically significant among the groups. In contrast, there was a significant increasing trend for Calf muscle thickness and Thigh-muscle cross-sectional area and thickness for the entire population. This finding can be attributed to the EMS treatment. No statistically significant differences were observed for most of the secondary outcomes (gait speed, body composition, autonomic nervous system activity, and blood chemistry) between any of the three treatment groups.

In contrast, at week 12 there was a statistically significant difference for the knee extension strength between W-BIO group and WHEY group (4.17 kg [95% confidence interval (CI) 0.41 - 7.94], p=0.0308) and between W-BIO group and CHO group (5.89 kg [95% confidence interval (CI) 1.78 - 10.01], p=0.0063). For the right knee extension at week 12, there was a statistically significant difference between group 3 and group 1 (5.35 kg [95% confidence interval (CI) 1.13 - 9.57], p=0.0145). Moreover, this effect was observed in males but not in females. These data suggest that a double approach combining EMS and specific dietary supplementation have a positive influence on muscle strength, and may improve the quality of life of elderly people. Thus, combining EMS and specific dietary intervention may be considered as a new method of treatment of lifestyle related diseases caused by aging and lack of exercise.

METHODS

The primary objective was to determine the efficacy of the intake of a whey-based supplement in addition to electrical muscle stimulation (EMS) for improvement of muscle morphology and functionality of frail individuals during 12 weeks of EMS and nutritional intervention. This was a pilot study, parallel, double-blind, randomized study design, and single center with 3 groups (see below).

Subjects:

Forty-one subjects initially volunteered for this study were all frail elderly people (age 65-90 years) presenting a frailty status according to the long-term care insurance (LCTI) system of Japan. All subjects included in the study were "free-living" people without any support or classified in Care support level 1, Care support level 2, Long-term care level 1 (LTC1), and Long-term care level 2 (LTC2) according to LTCI system. Subject with gait speed between 0.6 and 1.2m/s were included. All subjects were screened by a physician and a care manager, asking orthopedic and medical questions, to determine their suitability to participate in exercise interventions including electrical muscle stimulation.

Subjects were randomized in a 1:1:1 ratio to one of the three study groups; Isocaloric (95kcal) beverage containing either (A) 20g of carbohydrate (maltodextrin glucose syrup 21DE) + placebo capsules (CHO) (n=13), (B) 20g of whey protein isolate (Prolacta 95) + placebo capsules (WHEY) (n=15), or (C) 20g of whey protein isolate (Prolacta 95) plus rutin capsules (500mg rutin per day)+ w3-FA/curcumin capsules (daily doses: 500mg curcumin and 1.5g w3-FA type NAD supplied by Sofinol) (W-BIO) (n=13). Four subjects (1 in CHO group and 3 in W-BIO group) were excluded from the per-protocol analysis population due to subjects not compliant with the inclusion criteria (n=2) or prematurely withdrawn from follow-up (n=2).

Depending on group assignment, subjects ingested orally 1 of 3 experimental beverages (A-C) dissolved in 220ml of water. On the days with EMS treatment (2 times a week), this beverage was given just after EMS (maximal stimulation of protein synthesis). To be able to discriminate the specific effect of the supplement (and not the one from the lunch), EMS was applied as soon as possible on the morning (so that the supplement will be taken at least 1 hour before the meal) or later in the afternoon (at least 2 hours after the end of the lunch). On the day without EMS treatment, subjects drank the dietary supplement at the same time they were used to drink it when they received EMS. The subjects also ingested 7 capsules per day, 2 during the breakfast, 3 during the lunch and 2 during the diner for the duration of the study: 2 hard capsule containing 500mg of rutin/day or placebo, 5 soft capsules containing a mix of fish oil (1.5g fish oil/day) and curcumin (500mg curcumin per day. All 41 subjects performed a Mini-Nutritional Assessment (MNA) to evaluate their basal nutritional state at baseline and after 12 weeks.

Electrical muscle stimulation (EMS) procedures:

Although EMS has undergone a decline in use, mainly because of stimulation discomfort, new technologies allow painless application of strong contractions. Such activation can be applied in higher exercise dosages and more efficiently than people are likely to achieve with exercise. The electrical muscle stimulation procedures have been fully described elsewhere (Hasegawa et al., 2011; Kimura et al., 2011; Miyamoto et al., 2012; Moritani et al., 2005). Briefly, all subjects received EMS training 2 times a week during 12 weeks. The subjects were asked to attach belt-type electrodes around the waist and both knees and ankles to stimulate inner muscles as well as the gluteus maximus muscle, the quadriceps femoris, the hamstrings, the triceps surae, and tibialis anterior muscles at 20 Hz (Muscle hypertrophy mode) of stimulus frequency. The stimulation intensity of EMS was regulated to the maximal tolerable level of each individual without discomfort. The EMS training was provided to the subjects for 20 min, 2 times per week for 12 weeks. Each time before the EMS, the physical conditions of the subjects were checked. EMS training was performed in a sitting position at rest to minimize the risk of developing lightheadedness, dizziness, falling, and fainting caused by rapid movement. A specially designed muscle stimulator (Auto Tens pro, Homer Ion Co. Ltd., Tokyo, Japan) was used for EMS training in this investigation. The stimulator current waveform was designed to produce co-contractions in the lower extremity muscle groups at a frequency of 20 Hz with a pulse width of 250 $\mu$s. The duty cycle was a 5 s stimulation with a 2 s pause for a period of 20 min. Moreover, an exponential climbing pulse was used to reduce discomfort during muscle stimulation.

The reasons and rationale of these EMS procedures just described above was the fact that high-frequency fatigue is evident when the active force is depressed at frequencies that previously elicited maximal force. High-frequency fatigue induces excessive loss of force, which can be due to electrical propagation failure with a rapid decline in the evoked action potential amplitude. During this period of high-frequency force fatigue, considerably greater force is generated at 20 Hz stimulation (Moritani et al., 1985). Thus, high-frequency fatigue could be largely accounted for by a failure of electrical transmission that may be due to reduced muscle membrane excitability leading to a reduction in the evoked potential amplitude and conduction time (Jones et al., 1979; Moritani et al., 1985).

Most of the previous studies reported the efficacy of EMS using very high-frequency (2500 Hz) or high-frequency stimulations (50 or 80 Hz). Eriksson et al. (1981) showed that muscle enzyme activities, fiber size, and mitochondrial properties in the quadriceps femoris did not change with 50 Hz EMS training sessions over 4-5 weeks. Thus, patients in previous studies employing high-frequency (50 or 80 Hz) EMS training might have suffered from the high-frequency fatigue, so that the intended muscles were not effectively contracted. This evidence indicates that 20 Hz EMS has the potential to elicit more effective muscular improvement (a combined adaptation of neural factors and morphological changes) than high-frequency (50 or 80 Hz) EMS.

Muscle morphology assessment:

Muscle thickness of the quadriceps femoris, hamstrings muscle group, and triceps surae muscle was assessed by using ultrasonography at baseline, 4 weeks, 8 weeks and 12 weeks of the experimental intervention. Strong correlations have been reported between muscle thickness measured by B-mode ultrasound and site-matched skeletal muscle mass measured by MRI (Dupont et al., 2001; Fukunaga et al., 2001). Therefore, it is plausible to use muscle thickness measurements to estimate muscle size and degree of muscle hypertrophy. Previous studies have shown the reliability of the ultrasound technique for measuring muscle thickness (Kellis et al., 2009; Reeves et al., 2004). Also, the reliability of the ultrasonographic measurement was measured in this study. The intraclass correlation coefficients in RF, VL, and CA were 0.97 (0.88-0.99), 0.96 (0.85-0.99), and 0.99 (0.96-1.0), respectively.

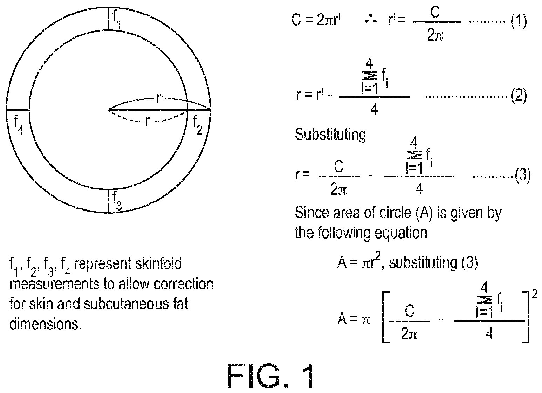

The measurement of muscle thickness by ultrasonography was standardized as follows: All scans were carried out at baseline and every four weeks after treatment. Each subject was examined by the same operator, using a real-time scanner (SSD-900, ALOKA, Tokyo, Japan) with a 5 MHz broadband transducer. A water-based gel was applied to the probe before the imaging procedure. During imaging, the transducer was held perpendicular to the surface of the skin and special care was taken to avoid excessive pressure. The measurement site was at the thickest part of the muscles with standardized procedures using skeletal markers carefully located. The imaging and measurements was performed unilaterally with the subjects in a sitting position for the quadriceps femoris and triceps surae muscles, respectively as these muscles were the main determinant for gait speed. The obtained images were stored on site and the entire data were analyzed later by using National Institute of Health (NIH) Image program. Each imaging data was analyzed in blind fashion as to the subject and date information in order to avoid any experimental bias. The measurement of muscle volume by ultrasonography was also standardized in the following way; in addition to the measurement of muscle thickness, the changes in thigh and calf muscle groups was estimated by measuring the circumferences of these muscle groups with standardized procedures. Subcutaneous fat thickness at four sites of each muscle group were determined by ultrasonography and averaged. Then, each muscle group volume was calculated algebraically by using the method of Moritani and deVries (1979). (see **FIG. 1**).

Autonomic nervous system activity evaluation:

Heart rate variability (HRV) power spectral analysis is a well-accepted, useful and noninvasive method, and has provided a comprehensive, quantitative and qualitative evaluation of neuroautonomic function under various research and clinical settings (Conny et al., 1993; Moritani et al., 1995). In general, power spectral analysis of HRV has shown at least two distinct regions of periodicity in electrocardiogram (ECG) R-R intervals. The high frequency component (> 0.15 Hz) is a major contributor to reflecting the parasympathetic nervous system (PNS) activity and the low frequency component (< 0.15 Hz) is associated with both the sympathetic nervous system (SNS) and the PNS activities (Akselrod et al., 1981; Moritani et al., 1993).

The R-R interval power spectral analysis procedures have been fully described elsewhere (Moritani et al., 1993; Matsumoto et al., 1999, 2001). Briefly, analog output of the ECG monitor (Life Scope, Nihon Kohden, Tokyo, Japan) was digitized via a 13-bit analog-to-digital converter (HTB 410; Trans Era, South Orem, UT) at a sampling rate of 1000 Hz. The digitized ECG signals were differentiated, and the resultant ECG QRS spikes and the intervals of the impulses (R-R intervals) were stored sequentially on a hard disk for later analyses. Before R-R spectral analysis was performed, the stored R-R interval data was displayed and aligned sequentially to obtain equally spaced samples with an effective sampling frequency of 2 Hz and displayed on a computer screen for visual inspection. Then, the direct current component and linear trend were completely eliminated by digital filtering for the band-pass between 0.03 and 0.5 Hz. The root mean square value of the R-R interval was calculated as representing the average amplitude. After passing through the Hamming-type data window, power spectral analysis by means of a fast Fourier transform was performed on a consecutive 256-second time series of R-R interval data obtained during the test. The spectral powers in frequency domain were quantified by integrating the areas under the curves for the following respective band width: the low frequency (LF: 0.03 and 0.15 Hz), an indicator of both SNS and PNS activity; the high frequency (HF: 0.15 and 0.5 Hz), which solely reflects the PNS activity; and the total power (TP: 0.03 and 0.5 Hz) representing the overall ANS activity, respectively.

Physical performance tests:

Muscle strength measurements. The procedures have been reported elsewhere (Watanabe et al., 2012a, 21012b). Briefly, isometric knee extensions were performed on a custom dynamometer mounting a force transducer (LU-100KSE; Kyowa Electronic Instruments, Tokyo, Japan). During contraction, both hip and knee joint angles were flexed at 90° (180° is fully extended), respectively. The maximal voluntary contraction (MVC) involved a gradual increase in knee extension force exerted by the knee extensor muscles from baseline to maximum in 2-3 s and then sustained at maximum for 2 s. The timing of the task was based on a verbal count given at a 1-s interval, with vigorous encouragement from the investigators when the force began to plateau. The subjects performed at least two MVC trials of each knee with 2 min rest between trials. The highest MVC force was used for comparison.

_Gait speed._ As a part of performance evaluation, gait speed was determined during screening, baseline before any EMS or supplement, and 12 weeks later, after the last EMS treatment and supplement, the 6-m walking time (seconds) along a 6-meter straight path marked with a tape on the floor, using a custom made trigger-linked stop-watch. The subjects were asked to walk normally as in daily life during assessments. Usual walking aids (e.g. stick, walker) were allowed. Gait speed was evaluated three times for each subject and averaged.

_Body composition._ Assessments of body composition (fat mass, lean mass, express as Kg and %) by using bioelectrical impedance analysis were carried out at baseline and 12 weeks of treatment. These measurements were performed for all the subjects by using most advanced bioelectrical impedance analysis device (Tanita BC-118D, Tanita Ltd., Tokyo, Japan), using hand-held leads together with flat foot sole electrodes with an excitation current of 500 microamperes at 50 KHz. The impedance value measured was used to calculate the lean mass and fat mass of Arms, Legs, Torso, and Whole body, respectively.

_Blood chemistry._ Blood was sampled from an antecubital vein into vacuum tubes after overnight fasting. Blood test for markers of insulin sensitivity (change in blood glucose, hemoglobin A1C, plasma insulin and C-peptide concentrations), red blood cell count, white blood cell count, plasma fatty acid profile, markers of inflammation (CRP, transthyretin, fibrinogen and orosomucoid), blood chemistry of CPK, HDL and LDL Cholesterol, albumin, total protein and triglycerides, respectively. Blood test of coagulation parameters (platelet count, prothrombin time and partial prothrombin time) were measured at baseline, 2, 6, 12 weeks to guarantee safety with the nutritional intervention. For the other parameters, measurements were performed at baseline and after 12 weeks of the intervention with EMS and nutrition supplementation.

Statistical analysis:

The primary analysis was performed on the Full Analysis Set (FAS) and Per-Protocol (PP) analysis populations comparing group 2 with group 1. The muscle thickness (in mm$^2$) was analyzed using a mixed model for repeated measures with baseline measurement, gender, age at randomization and time as covariates. The Least-Squares means per muscle location and treatment group at week 12 will be presented.

The O'Brien OLS test between group 1 and group 2 was used as a global test to evaluate the product effect at week 12 on both muscle locations. The O'Brien OLS statistic was determined as follows:

T-test statistic testing for the treatment effect at visit 8 for each of the muscle locations independently (i.e. Calf and thigh) is calculated

Then the OLS statistic was determined as:

$$t_{OLS} = \frac{j't}{\sqrt{j'\hat{A}j}}$$

where t is a vector of the t-test statistics of the Calf and Thigh measurements, j is a vector of 1, and Â is the sample correlation matrix of the original observations.

The approximation of degrees of freedom (d.f.) of the $t_{OLS}$ was defined as d.f.=0.5*(n$_1$+n$_2$-2)*(1+1/m$^2$) where m is the number of endpoints analyzed (i.e. calf + thigh) and n$_1$ and n$_2$ is the number of subjects in group 1 and group 2 respectively for whom the calf and the thigh measurements available at visit 8.

The secondary analyses were performed on the FAS population only. The analyses which were performed are detailed in section 11.2 of the SAP.

Mixed-effect linear models were used for longitudinal data in order to account for the correlation between repeated measures. Fixed effects linear models were used for analyzing the change from baseline to one time point. Multiple comparisons (W-BIO vs. PLACEBO and WHEY vs. PLACEBO) were carried out where deemed necessary. Adjusted p-values using the single-step adjustment were reported for these multiple comparisons. O'Brien's Global tests were performed to test directional hypothesis combining the effects on muscle cross-sectional area and thickness. This was done only to compare the W-BIO group vs. PLACEBO and separately for Calf and Thigh muscles. All data analysis was carried out using R, version 3.0.1.

RESULTS

Table 1 (below) represents population description of the parameters at baseline. The majority of subjects randomized in the trial were females. Subjects included in group W-BIO tended to be slightly heavier (BMI of 22.7 kg/m$^2$ vs. 20.3 and 21.3 in group CHO and WHEY respectively) and had a larger calf muscle (cross section area, mm$^2$) compared to subjects included in groups CHO and WHEY The right and left knee extension strength was inferior for subjects randomized to group CHO compared to measurements obtained for subjects randomized to groups WHEY and W-BIO respectively.

Table 1. Population description of the parameters at baseline.

|  | CHO (N=13) | WHEY (N=15) | WHEY-BIO (N=13) |
|---|---|---|---|
| **Age at randomization (years)** | 75.9 (8.7) | 78.0 (4.9) | 76.6 (7.3) |
| **Female** | 11 (84.6%) | 12 (80.0%) | 10 (76.9%) |
| **Body height (cm)** | 151.62 (7.49) | 152.40 (9.42) | 152.77 (8.09) |
| **Body weight (kg)** | 46.98 (10.82) | 49.69 (10.67) | 53.05 (8.81) |
| **Body Mass Index (kg/m$^2$)** | 20.3 (3.4) | 21.3 (3.5) | 22.7 (3.0) |
| **Thigh (cross section area, mm2)** | 11737.38 (2673.74) | 12213.68 (3190.19) | 12033.29 (2258.83) |
| **Calf (cross section area, mm$^2$)** | 6699.18 (1163.74) | 6853.66 (1767.15) | 7598.23 (1080.07) |
| **Gait speed (m/s)** | 1.20 (0.32) | 1.12 (0.33) | 1.15 (0.45) |
| **Right knee extension strength (kg)** | 20.09 (4.37) | 25.46 (9.59) | 25.03 (13.28) |
| **Left knee extension strength (kg)** | 18.76 (5.53) | 24.85 (8.21) | 22.04 (11.16) |
| Data are mean (SD); N=Number of subjects, %=percentage; SD=standard deviation; min=minimum; max=maximum; perc=percentile | | | |

Muscle morphology:

Longitudinal analysis of muscle surface area and thickness. Global test was performed to compare the W-BIO group vs. PLACEBO in terms of Calf cross-sectional area (CSA) and thickness and CSA taken together and similarly for Thigh. No statistical significance was found for these comparisons.

Effect of dietary treatment on muscle surface area and thickness at the end of the intervention. When the different groups were compared at the end of the treatment period (after 3 months) no statistically significant difference among three groups with respect to the thigh and calf muscle thickness or cross sectional area were found. There were no statistically significant differences between any of the study treatment groups for any of the time points with respect to the thigh and calf muscle cross sectional area. Thus, considering together results of muscle morphology, they show that the treatment induced an increase in muscle surface area and thickness which was observed in the three groups and thus was probably more related to EMS treatment than to specific nutritional intervention.

Physical Performance:

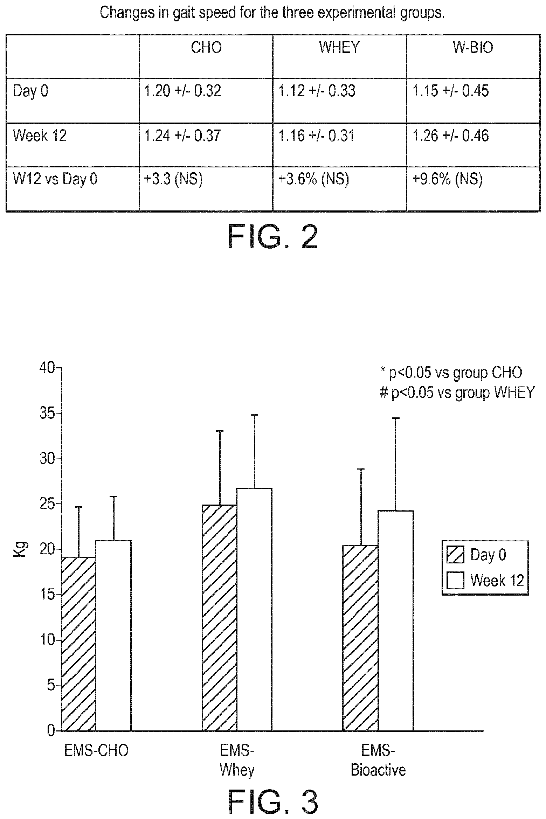

Gait speed. Table 2 shows the descriptive results for the gait speed (m/s) at the baseline and at week 12 for the three treatment groups (FIG. 2). The largest improvement in gait speed was seen for the W-BIO group from 1.15 $\pm$ 0.45 to 1.26 $\pm$ 0.46 m/s (nearly 9.5% increase). Only moderate increase in the gait speed was seen for the CHO group (1.20 $\pm$ 0.32 to 1.24 $\pm$ 0.37 m/s (3.3% increase) and WHEY group (1.12 $\pm$ 0.33 to 1.16 $\pm$ 0.31 m/s (3.6% increase), respectively. However, these differences did not reach the statistical significance.

Muscle strength. **FIG. 3** represents the changes of the left knee extension muscle strength at the baseline and at week 12 for the three treatment groups. At week 12, there was a statistically significant difference between W-BIO group and WHEY group (4.17 kg [95% confidence interval (CI) 0.41 - 7.94], p=0.0308) and between W-BIO group and CHO group (5.89 kg [95% confidence interval (CI) 1.78 - 10.01], p=0.0063) for the left knee extension.

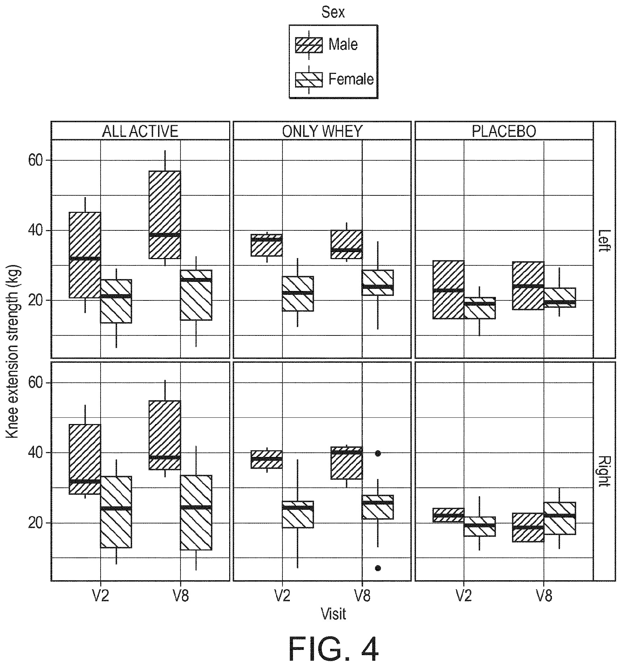

Moreover, mixed-effects models were fitted in order to take into account the correlation between the two measurements taken on the two knees for each subject at each time point. Results (see **FIG. 4**) indicate that there is a statistically significant interaction between treatment and gender. In other words the treatment effect differs over the two genders. This is highlighted in terms of comparisons between the active treatment groups (also called W-BIO) vs. Placebo (CHO) separately for males and females. Thus the treatment appears to have induced a strong improvement of knee extension strength in male from group W-BIO but this effect was not observed in women.

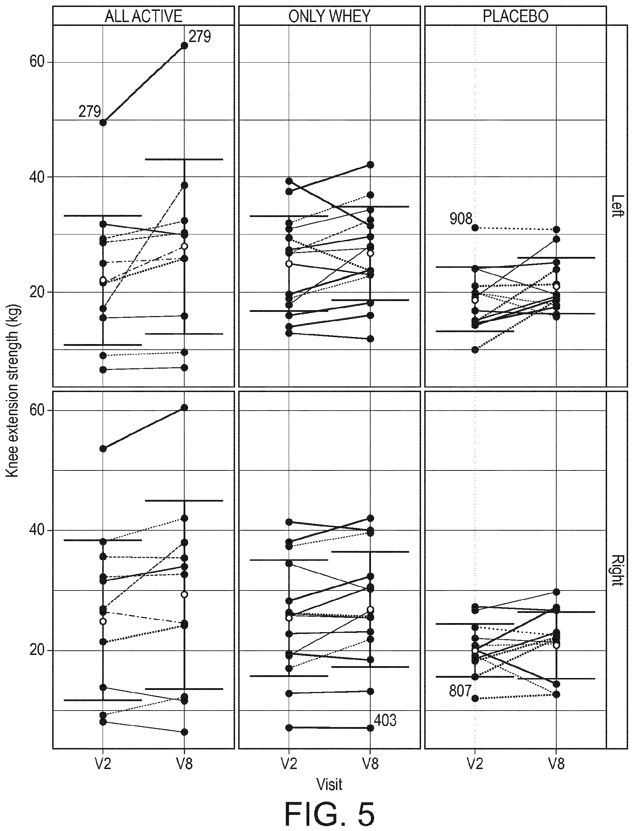

Finally, the results when each subject is represented individually are interesting **(FIG. 5)**: nearly all subjects from W-BIO presented a positive slope between V2 and V8 (whatever the gender) when the evolution appears to go up and down in the 2 other groups. This presentation give therefore a direct visualization of the specific benefit observed in the W-BIO group and demonstrated by the statistical analysis described above.

Body composition. Anthropometric measurements (fat mass, lean mass, express as Kg and %) by using bioelectrical impedance analysis were carried out at baseline and 12 weeks of experimental treatment. There were no statistically significant differences between any of the study treatment groups at the baseline and at week 12.

Autonomic nervous system activity. Electrocardiography R-R interval power spectral analyses revealed no statistically significant differences in resting heart rate, LF (sympathetic nervous system activity), HF (parasympathetic nervous system activity) and TP (overall autonomic nervous system activity) between any of the study treatment groups at the baseline and at week 12.

Blood Analyses. There were no statistically significant differences between any of the study treatment groups for the blood test results for markers of insulin sensitivity (change in blood glucose, hemoglobin A1C, plasma insulin and C-peptide concentrations), red blood cell count, white blood cell count, plasma fatty acid profile, markers of inflammation (CRP, transthyretin, fibrinogen and orosomucoid), blood chemistry of CPK, HDL and LDL Cholesterol, albumin, total protein and triglycerides at baseline and at week 12. Similarly, blood test of coagulation parameters (platelet count, prothrombin time and partial prothrombin time) measured at baseline, 2, 6, 12 weeks demonstrated no significant group differences.

DISCUSSION

The study demonstrated that knee extension strength of frail individuals was significantly increased (18.8% for left leg and 7.3% for the right leg) in the W-BIO group than other two groups (CHO and WHEY) after 12 weeks of bioactive supplement (whey protein, rutin, w3-FA, and curcumin) together with EMS training. Also this W-BIO group demonstrated the largest improvement in the gait speed (9.6%) among the groups with carbohydrate or whey protein supplementation together with EMS training. Interestingly, this benefit on muscle strength was not related to an increase in muscle size. Indeed, a small increase of muscle size was observed in the 3 groups along the 12 weeks of treatment but this effect was similar in the 3 groups and was therefore probably specific to EMS treatment (and not related to dietary treatment).

Interestingly, a gender difference in the degree of response to EMS training was found, specifically male subjects demonstrating significant improvements in muscle thickness while no such significant changes was observed for the females.

The findings support the importance of "neural factors," which although not yet well-defined, certainly contribute to the display of maximal muscle force called strength. Human voluntary strength is determined not only by the quantity (muscle cross sectional area) and quality (muscle fiber types) of the involved muscle mass, but also by the extent to which the muscle mass has been activated (neural factors). Moreover, muscle quality is also related to intramuscular lipid inclusion, which increases with age and is responsible for a low muscle quality. Thus, the modification of muscle quality along the study and particularly to the benefit seen on W-BIO treatment group could be a decrease in lipid content in muscle together with an increase of muscle fiber number or size, thus allowing to get an improvement in strength, without any modification of muscle thickness or CSA (counterbalance between lipid and protein content).

In the present study, all three groups received the same EMS training that induced "involuntary" contractions of the major lower extremity muscle groups 2 times per week for a period of 12 weeks. In the absence of no significant differences in thigh and calf muscle thickness and estimated cross-sectional area among the three groups suggest the possibility that the W-BIO group might have gained the ability to activate the muscles to a higher extent by enhanced central motor drive and/or changed muscle fiber composition to develop more tension per cross-sectional area made possible by bioactive supplementation. The W-BIO group might have enhanced central nervous system integrity resulting in higher motor command to activate muscles.

Another possibility to explain the increased muscle strength observed specifically in W-BIO group would be that the polyphenols may help to manage the oxidative stress that is known to occur in inactive elderly persons. The polyphenols given together with protein may help to counteract anabolic resistance (by managing the oxidative stress) and thus could restore the anabolic effect of nutrients and proteins on muscle protein synthesis. In the same way, the EPA supplementation may also have induced an improvement in insulin sensitivity and thus stimulated muscle protein synthesis associated with whey proteins.

**Claims**

1. A composition comprising a protein source and an antioxidant for use in reducing a loss of muscle functionality in an elderly male, increasing muscle functionality in an elderly male after muscle atrophy, and/or improving recovery of muscle functionality after muscle atrophy in an elderly male, wherein the composition comprises a fatty acid, preferably an n-3 fatty acid, and, wherein the antioxidant comprises a polyphenol, wherein the polyphenol is selected from the group consisting of curcumin, rutin, quercetin and combinations thereof, and wherein the protein source comprises whey protein.

2. The composition for use according to claim 1, wherein the protein source comprises a protein selected from the group consisting of casein, pea protein, soy protein and combinations thereof.

3. The composition for use according to claim 1, wherein the composition is administered at least twice a week for a time period of at least one month.

4. The composition for use according to claim 1, wherein the muscle functionality comprises a characteristic selected from the group consisting of muscle strength, gait speed, and combinations thereof.

5. The composition for use according to claim 1, wherein the composition is administered in an amount that provides 0.1 to 0.4 g of the protein source per kg body weight of the elderly male per day.

6. The composition for use according to claim 1, wherein the composition is administered in an amount that provides 0.01 to 0.04 g of leucine per kg body weight of the elderly male per day.

7. The composition for use according to claim 1, wherein the elderly male has sarcopenia.

8. A composition comprising a protein source, and an antioxidant in an amount that is therapeutically effective, for use in (i) treating sarcopenia in an elderly male having sarcopenia, (ii) preventing sarcopenia in an elderly male, or (iii) reducing a loss of muscle functionality in an elderly male having sarcopenia, wherein the composition comprises a fatty acid, preferably an n-3 fatty acid, and, wherein the antioxidant comprises a polyphenol, wherein the polyphenol is selected from the group consisting of curcumin, rutin, quercetin and combinations thereof, and wherein the protein source comprises whey protein.

9. The composition for use according to any of preceding claims wherein the protein source comprises leucine, and the leucine is present in the composition up to an amount of 10 wt%.

10. The composition for use according to any of preceding claims wherein the composition is selected from the group consisting of food compositions, dietary supplements, nutritional compositions, nutraceuticals, powdered nutritional products to be reconstituted in water or milk before consumption, food additives, medicaments, drinks, and combinations thereof.

**Patentansprüche**

1. Zusammensetzung, umfassend eine Proteinquelle und ein Antioxidationsmittel zur Verwendung bei einem Verringern eines Verlusts von Muskelfunktionalität in einem älteren Mann, einem Steigern von Muskelfunktionalität in einem älteren Mann nach einer Muskelatrophie und/oder einem Verbessern einer Wiederherstellung von Muskelfunktionalität nach der Muskelatrophie in einem älteren Mann, wobei die Zusammensetzung eine Fettsäure, vorzugsweise eine n-3-Fettsäure, umfasst und wobei das Antioxidationsmittel ein Polyphenol umfasst, wobei das Polyphenol aus der Gruppe ausgewählt ist, bestehend aus Curcumin, Rutin, Quercetin und Kombinationen davon, und wobei die Proteinquelle Molkeprotein umfasst.

2. Zusammensetzung zur Verwendung nach Anspruch 1, wobei die Proteinquelle ein Protein umfasst, das aus der Gruppe ausgewählt ist, bestehend aus Molkeprotein, Kasein, Erbsenprotein, Sojaprotein und Kombinationen davon.

3. Zusammensetzung zur Verwendung nach Anspruch 1, wobei die Zusammensetzung mindestens zweimal pro Woche für einen Zeitraum von mindestens einem Monat verabreicht wird.

4. Zusammensetzung nach Anspruch 1, wobei die Muskelfunktionalität eine Eigenschaft umfasst, die aus der Gruppe ausgewählt ist, bestehend aus Muskelkraft, Ganggeschwindigkeit und Kombination davon.

5. Zusammensetzung zur Verwendung nach Anspruch 1, wobei die Zusammensetzung in einer Menge verabreicht wird, die 0,1 g bis 0,4 g der Proteinquelle pro kg Körpergewicht des älteren Mannes pro Tag bereitstellt.

6. Zusammensetzung zur Verwendung nach Anspruch 1, wobei die Zusammensetzung in einer Menge verabreicht wird, die 0,01 g bis 0,04 g des Leucins pro kg Körpergewicht des älteren Mannes pro Tag bereitstellt.

7. Zusammensetzung zur Verwendung nach Anspruch 1, wobei der ältere Mann Sarkopenie aufweist.

8. Zusammensetzung, umfassend eine Proteinquelle, und ein Antioxidationsmittel in einer Menge, die therapeutisch wirksam ist, zur Verwendung bei (i) dem Behandeln von Sarkopenie in einem älteren Mann, der Sarkopenie aufweist, (ii) einem Verhindern von Sarkopenie in einem älteren Mann oder (iii) Reduzieren eines Verlusts der Muskelfunktionalität in einem älteren Mann, der Sarkopenie aufweist, wobei die Zusammensetzung eine Fettsäure, vorzugsweise eine n-3 Fettsäure, umfasst und wobei das Antioxidationsmittel ein Polyphenol umfasst, wobei das Polyphenol aus der Gruppe ausgewählt ist, bestehend aus Curcumin, Rutin, Quercetin und Kombinationen davon, und wobei die Proteinquelle Molkeprotein umfasst.

9. Zusammensetzung zur Verwendung nach einem der vorstehenden Ansprüche, wobei die Proteinquelle Leucin umfasst und das Leucin in der Zusammensetzung bis zu einer Menge von 10 Gew.-% vorhanden ist.

10. Zusammensetzung zur Verwendung nach einem der vorstehenden Ansprüche, wobei die Zusammensetzung aus der Gruppe ausgewählt ist, bestehend aus Nahrungsmittelzusammensetzungen, Nahrungsergänzungsmitteln, Nährstoffzusammensetzungen, Nutrazeutika, pulverisierten Nährstoffprodukten, die vor einem Verzehr in Wasser oder Milch aufzulösen sind, Lebensmittelzusätzen, Medikamenten, Getränken und Kombinationen davon.

## Revendications

1. Composition comprenant une source de protéines et un antioxydant pour une utilisation dans la réduction d'une perte de fonctionnalité musculaire chez un homme âgé, l'augmentation de la fonctionnalité musculaire chez un homme âgé après une atrophie musculaire, et/ou l'amélioration de la récupération de la fonctionnalité musculaire après une atrophie musculaire chez un homme âgé, la composition comprenant un acide gras, de préférence un acide gras n-3, et l'antioxydant comprenant un polyphénol, le polyphénol étant choisi dans le groupe constitué de curcumine, rutine, quercétine et de combinaisons de celles-ci, et la source de protéine comprenant une protéine de lactosérum.

2. Composition pour une utilisation selon la revendication 1, dans laquelle la source de protéine comprend une protéine choisie dans le groupe constitué de caséine, protéine de pois, protéine de soja et de combinaisons de celles-ci.

3. Composition pour une utilisation selon la revendication 1, dans laquelle la composition est administrée au moins deux fois par semaine pendant une période d'au moins un mois.

4. Composition pour une utilisation selon la revendication 1, dans laquelle la fonctionnalité musculaire comprend une caractéristique choisie dans le groupe constitué de force musculaire, vitesse de marche et de combinaisons de celles-ci.

5. Composition pour une utilisation selon la revendication 1, dans laquelle la composition est administrée en une quantité qui fournit 0,1 à 0,4 g de la source de protéines par kg de poids corporel de l'homme âgé par jour.

6. Composition pour une utilisation selon la revendication 1, dans laquelle la composition est administrée en une quantité qui fournit 0,01 à 0,04 g de leucine par kg de poids corporel de l'homme âgé par jour.

7. Composition pour une utilisation selon la revendication 1, dans laquelle l'homme âgé souffre de sarcopénie.

8. Composition comprenant une source de protéines, et un antioxydant en une quantité efficace sur le plan thérapeutique, pour une utilisation dans (i) le traitement de la sarcopénie chez un homme âgé souffrant de sarcopénie, (ii) la prévention de la sarcopénie chez un homme âgé, ou (iii) la réduction d'une perte de fonctionnalité musculaire chez un homme âgé souffrant de sarcopénie, la composition comprenant un acide gras, de préférence un acide gras n-3, et, l'antioxydant comprenant un polyphénol, le polyphénol étant choisi dans le groupe constitué de curcumine, rutine, quercétine et de combinaisons de celles-ci, et la source de protéine comprenant une protéine de lactosérum.

9. Composition pour une utilisation selon l'une quelconque des revendications précédentes, dans laquelle la source de protéine comprend de la leucine, et la leucine est présente dans la composition jusqu'à une quantité de 10 % en poids.

10. Composition pour une utilisation selon l'une quelconque des revendications précédentes, dans laquelle la composition est choisie dans le groupe constitué de compositions alimentaires, compléments alimentaires, compositions nutritionnelles, produits nutraceutiques, produits nutritionnels en poudre destinés à être reconstitués dans de l'eau ou du lait avant consommation, additifs alimentaires, médicaments, boissons et de combinaisons de ceux-ci.

$f_1, f_2, f_3, f_4$ represent skinfold measurements to allow correction for skin and subcutaneous fat dimensions.

$$C = 2\pi r^I \quad \therefore \quad r^I = \frac{C}{2\pi} \quad \cdots\cdots (1)$$

$$r = r^I - \frac{\sum_{i=1}^{4} f_i}{4} \quad \cdots\cdots (2)$$

Substituting

$$r = \frac{C}{2\pi} - \frac{\sum_{i=1}^{4} f_i}{4} \quad \cdots\cdots (3)$$

Since area of circle (A) is given by the following equation

$A = \pi r^2$, substituting (3)

$$A = \pi \left[ \frac{C}{2\pi} - \frac{\sum_{i=1}^{4} f_i}{4} \right]^2$$

# FIG. 1

Changes in gait speed for the three experimental groups.

|  | CHO | WHEY | W-BIO |
|---|---|---|---|
| Day 0 | 1.20 +/- 0.32 | 1.12 +/- 0.33 | 1.15 +/- 0.45 |
| Week 12 | 1.24 +/- 0.37 | 1.16 +/- 0.31 | 1.26 +/- 0.46 |
| W12 vs Day 0 | +3.3 (NS) | +3.6% (NS) | +9.6% (NS) |

# FIG. 2

# FIG. 3

FIG. 4

FIG. 5